Europäisches Patentamt

(19) European Patent Office

Office européen des brevets

(11) Veröffentlichungsnummer : **0 008 396**
B2

# (12) NEUE EUROPÄISCHE PATENTSCHRIFT

(45) Veröffentlichungstag der neuen Patentschrift :
27.05.87

(51) Int. Cl.⁴ : **C 07 C 53/12, C 07 C 51/54**

(21) Anmeldenummer : 79102790.7

(22) Anmeldetag : 03.08.79

(54) **Verfahren zur Herstellung von Essigsäureanhydrid.**

(30) Priorität : **17.08.78 DE 2836084**

(43) Veröffentlichungstag der Anmeldung :
**05.03.80 Patentblatt 80/05**

(45) Bekanntmachung des Hinweises auf die Patenterteilung : **30.09.81 Patentblatt 81/39**

(45) Bekanntmachung des Hinweises auf die Entscheidung über den Einspruch : **27.05.87 Patentblatt 87/22**

(84) Benannte Vertragsstaaten :
**BE DE FR GB IT NL**

(56) Entgegenhaltungen :
EP-A- 0 079 461
EP-A- 0 081 152
DE-A- 2 450 965
DE-A- 2 610 035
DE-A- 2 610 036
DE-A- 2 658 216
DE-A- 3 148 006
DE-C- 933 148
DE-C- 2 462 910
**Kirk-Othmer, Encyclopedia of Chemical Technology, (1968) Seiten 859-865**
**E.H. Rodd, Chemistry of Carbon Compounds, Bd. IV (1957) Seite 504**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Erpenbach, Heinz, Dr.**
**Oberbuschweg 22**
**D-5000 Köln 50 (DE)**
Erfinder : **Gehrmann, Klaus, Dr.**
**Geschwister-Scholl-Strasse 32**
**D-5042 Erftstadt (DE)**
Erfinder : **Kübbeler, Hans-Klaus, Dr.**
**Bünnagelring 31**
**D-5557 Swisttal 8 (DE)**
Erfinder : **Schmitz, Klaus**
**Rodderweg 46**
**D-5042 Erftstadt (DE)**

EP 0 008 396 B2

## Beschreibung

Die Erfindung betrifft ein Verfahren zur Herstellung von Essigsäureanhydrid durch Umsetzung von Methylacetat und/oder Dimethyläther mit Kohlenmonoxid unter praktisch wasserfreien Bedingungen bei Temperaturen von 350 bis 575 K und Drücken von 1 bis 300 bar in Gegenwart eines Katalysatorsystems aus Edelmetallen der Gruppe VIII des Periodensystems der Elemente oder deren Verbindungen und Jod und/oder dessen Verbindungen sowie in Gegenwart einer aliphatischen Carbonsäure mit 1-8 Kohlenstoffatomen, welches dadurch gekennzeichnet ist, daß man als Promotor ausschließlich heterocyclische aromatische Verbindungen, in denen mindestens ein Heteroatom ein quaternäres Stickstoffatom ist und deren Schmelz- oder Mischschmelzpunkt unterhalb 413 K, dem Siedepunkt von Essigsäureanhydrid, liegt, einsetzt.

Die DE-A1-26 58 216 beschreibt bereits ein vergleichbares Verfahren zur Herstellung von Monocarbonsäureanhydriden, jedoch mit dem Unterschied, daß dort anstelle des Edelmetalls Nickel und Chrom eingesetzt werden. Als Löse- oder Verdünnungsmittel kann eine Carbonsäure, z. B. Essigsäure, dienen, doch arbeiten sämtliche 26 Beispiele ohne Carbonsäure. Gemäß vorliegender Erfindung ist die Anwesenheit einer Carbonsäure hingegen zwingend erforderlich, da sie als Promotor im Katalysatorsystem wirkt.

Die DE-A1-26 58 216 verwendet weiterhin eine Organostickstoff- oder Organophosphorverbindung mit dreiwertigem Stickstoff bzw. Phosphor als Promotor. Von den 26 Beispielen können nur die Beispiele 1, 2 und 7 zum Vergleich herangezogen werden, da die übrigen Beispiele mit nichtheterozyklischen und/oder nicht-aromatischen stickstoffhaltigen Promotoren oder mit Organophosphorverbindungen arbeiten.

In den Beispielen 1, 2 und 7 der DE-A1-26 58 216 wird neben einer heterozyklischen, aromatischen Organostickstoffverbindung auch Methyljodid eingesetzt. Es ist jedoch an keiner Stelle davon die Rede, daß sich daraus unter Quaternisierung eines Stickstoffatoms im aromatischen Heterozyklus Addukte bilden. Für eine solche Annahme sind die Zusammensetzungen der Katalysatorsysteme zu verschieden von denen vorliegender Erfindung. Dagegen spricht auch der Passus auf Seite 13, letzter Absatz der DE-A1-26 58 216, wo davon die Rede ist, daß sich die Promotorkomponenten durch Destillation des Reaktionsgemisches zurückgewinnen lassen.

Dies ist aber keinesfalls bei salzartigen Addukten aus heterozyklischen aromatischen Verbindungen mit einem quaternären N-Atom als Heteroatom und Methyljodid möglich, da diese undestillierbar sind, sondern nur bei Organostickstoffverbindungen mit dreibindigem Stickstoff, welche im Katalysatorsystem der DE-A1-26 58 216 keine Addukte bilden und destilliert werden können. Bei dem Verfahren vorliegender Erfindung ist eine solche Arbeitsweise unmöglich, da bei der destillativen Aufarbeitung die heterozyklische, quaternäre Ammoniumverbindung immer im Sumpf zurückbleibt und als Schmelze zurückgeführt werden muß.

Die DE-A1-26 10 036 beschreibt ebenfalls ein vergleichbares Verfahren zur Herstellung von Monocarbonsäureanhydriden, jedoch mit dem Unterschied, daß neben dem Edelmetall der Gruppe VIII des Periodensystems und einem Jodid ein Mehrfachpromotor eingesetzt wird, der ein Metall, vorzugsweise Chrom, Eisen, Kobalt, Nickel, sowie eine Organostickstoffverbindung oder Organophosphorverbindung mit dreibindigem Stickstoff bzw. Phosphor enthält.

Beim Verfahren der DE-A1-26 10 036 wirkt es sehr störend, daß die Metallverbindungen und Folgeprodukte des Mehrfachpromotors in siedendem Acetanhydrid weitgehend unlöslich sind, so daß die beim kontinuierlichen Verfahren erforderliche Kreislaufführung des Katalysator- und Promotorsystems sehr erschwert, wenn nicht gar unmöglich gemacht wird. Ebenso beeinträchtigen diese unlöslichen Verbindungen die Trennung des Acetanhydrids vom Katalysator- und Promotorsystem in unzulässigem Maße. Als Folge davon müßte eine langwierige, verlustreiche Zwischenbehandlung des teuren, edelmetallhaltigen Katalysators vorgenommen werden, wobei die auftretenden Katalysatorverluste zu einer schnellen Aktivitätsminderung im gesamten System führen. Diese Umstände verhinderten bisher die Durchführung des Verfahrens im technischen Maßstab.

Die eingangs geschilderte Erfindung vermeidet diese Nachteile, wobei überraschenderweise festgestellt wurde, daß auf den Einsatz der schwerlöslichen Metallsalze, z. B. von Chrom, im Promotorgemisch verzichtet werden kann, sofern anstelle der Organostickstoffverbindung oder Organophosphorverbindung mit dreibindigem Stickstoff bzw. Phosphor eine heterocyclische aromatische Verbindung mit quaternärem Stickstoff eingesetzt wird. Derartige Addukte mit quaternärem Stickstoff sind unter Reaktionsbedingungen schmelzflüssig und stören die Kreislaufführung des Katalysatorsystems in keiner Weise. Ebensowenig wird durch diesen Austausch die Selektivität des Katalysatorsystems herabgesetzt, vielmehr erhöhen die erfindungsgemäßen Maßnahmen die Aktivität des Katalysatorsystems beträchtlich. Die erfindungsgemäß eingesetzten heterocyclischen aromatischen Verbindungen mit mindestens einem quaternären Stickstoffatom als Heteroatom bilden einzeln oder im Gemisch sowohl unter Reaktionsbedingungen als auch unter den Bedingungen der Aufarbeitung der Reaktionsprodukte der Carbonylierung von Methylacetat bzw. Dimethyläther Schmelzen, die sich als geeignete Lösemittel für die Edelmetallkomplexe erweisen und darüber hinaus mit Essigsäureanhydrid gut mischbar sind.

Addukte mit diesen Eingenschaften sind z. B.

a) N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid ;

b) Pyridiniumacetat, N-Methylimidazoliumacetat, 3-Picoliniumacetat, 2,4-Lutidiniumacetat, 3,4-Lutidiniumacetat.

Die Promotoreigenschaft dieser Addukte wird durch die Anwesenheit einer aliphatischen Carbonsäure mit 1-8 Kohlenstoffatomen erheblich verstärkt.

Weiterhin kann die Erfindung bevorzugt und wahlweise dadurch gekennzeichnet sein, daß man

a) die heterocyclischen Verbindungen in Form ihrer Addukte mit Essigsäure oder Methyljodid einsetzt ;

b) das Katalysator- und Promotorsystem Edelmetall (-verbindung)/Jod(-verbindung)/Carbonsäure/heterocyclische Verbindung im Atom- bzw. Molverhältnis 1 : (1-1 400) : (10-2 000) : (1-1 200) einsetzt ;

c) Kohlenmonoxid/Wasserstoffgemische mit bis zu 10 Volum % Wasserstoff einsetzt.

Bevorzugt wird das Verfahren der Erfindung bei Temperaturen von 400-475 K und Drucken von 20 bis 150 bar durchgeführt. Je Mol Methylacetat und/oder Dimethyläther setzt man vorzugsweise 0,000 1 bis 0,01 Mol des Edelmetalls der Gruppe VIII des Periodensystems der Elemente oder seiner Verbindungen ein. Vorzugsweise setzt man das Katalysator- und Promotorsystem Edelmetall (-verbindung)/Jod (-verbindung)/Carbonsäure/heterocyclische Verbindung im Atom- bzw. Molverhältnis 1 : (10-300) : (25-600) : (10-300) ein. Der Einsatz von Essigsäure als Carbonsäure wird bevorzugt.

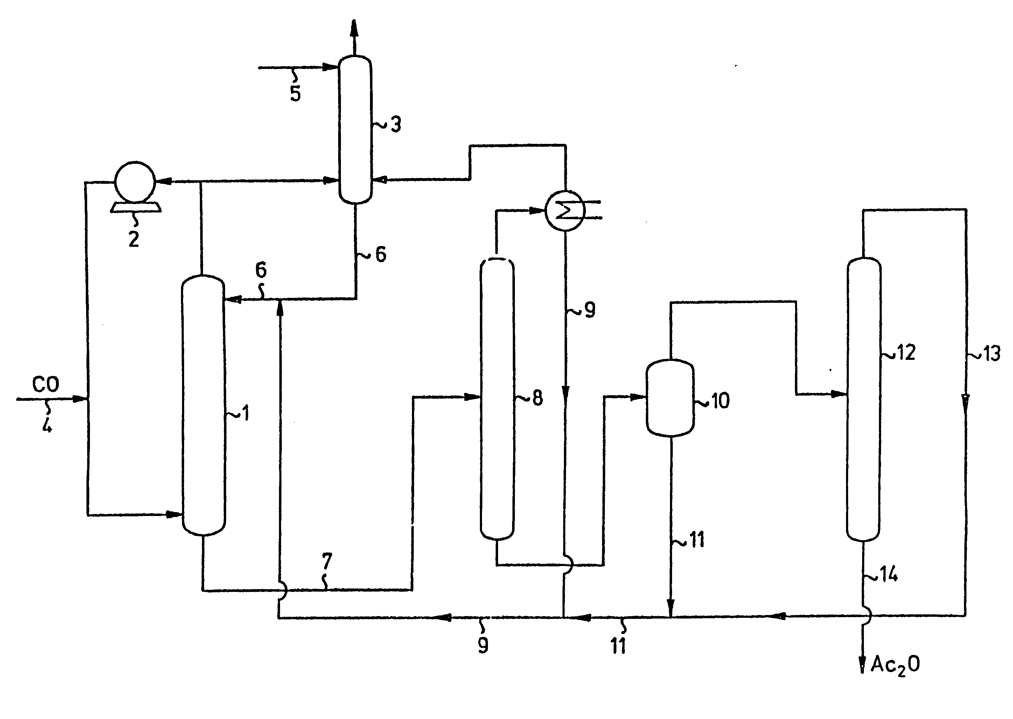

Ein mögliches Fließschema zur Durchführung des erfindungsgemäßen Verfahrens ist in der Zeichnung schematisch dargestellt und im folgenden beschrieben :

In einem Druckreaktor 1 aus Hastelloy C(R) werden Methylacetat und/oder Dimethyläther und Kohlenmonoxid oder ein Gemisch aus CO und $H_2$ mit bis zu 10 Vol.% $H_2$ in Gegenwart eines Katalysatorsystems aus Edelmetallen der Gruppe VIII des Periodensystems der Elemente oder deren Verbindungen und Jod und/oder dessen Verbindungen, vorzugsweise Methyljodid, sowie in Gegenwart einer Carbonsäure, vorzugsweise Essigsäure, und mindestens einer heterocyclischen aromatischen Verbindung, in der mindestens ein Heteroatom ein quaternäres Stickstoffatom ist, als Promotoren unter einem Druck von vorzugsweise 20 bis 150 bar und einer Temperatur von vorzugsweise 400 bis 475 K zu Acetanhydrid umgesetzt. Nichtumgesetztes Kohlenmonoxid und ggf. Wasserstoff werden über eine Gasumwälzpumpe 2 im Kreislauf geführt, während ein geringer Teilstrom das System als Abgas über eine Wäsche 3 verläßt. Frisches, ggf. wasserstoffhaltiges Kohlenmonoxid wird dem Umsatz entsprechend über Leitung 4 dem Kreislaufgas zudosiert. Die dem Umsatz entsprechende Menge frischen Methylacetats und/oder Dimethyläthers wird über Leitung 5 am Kopf der Wäsche 3 aufgegeben und über Leitung 6 dem Reaktor 1 zugeleitet. Das Reaktionsgemisch strömt über Leitung 7 aus dem Reaktor 1 ab. In der Destillationskolonne 8 erfolgt die Abtrennung der Leichtsieder (Methylacetat bzw. Dimethyläther, Methyljodid), die über Leitung 9 und 6 dem Reaktor 1 wieder zugeführt werden. Der Sumpf der Leichtsiederkolonne 8 wird im Verdampfer 10 in Destillat und Katalysator zerlegt. Letzterer gelangt über die Leitungen 11, 9 und 6 zum erneuten Einsatz in den Reaktor 1. Das Destillat des Verdampfers 10 wird in der Destillationskolonne 12 in Essigsäure, die über die Leitungen 13, 11, 9 und 6 in den Reaktor 1 zurückfließt, und in das hergestellte Acetanhydrid, das über Leitung 14 abgenommen wird, getrennt.

Beispiel 1

250 g Methylacetat, 1,6 g $RhCl_3 \cdot 3H_2O$, 50 g $CH_3J$, 50 g Essigsäure und 60 g N-Methyl-3-picoliniumjodid werden in einem Hastelloy-Autoklaven mit CO bei 40 bar und 450 K umgesetzt. Nach 45 min Reaktionszeit ergibt die Analyse die Bildung von 271 g Essigsäureanhydrid, entsprechend 578 g $Ac_2O$/g Rh.h.

Beispiel 2

250 g Methylacetat, 1,6 g $RhCl_3 \cdot 3H_2O$, 100 g $CH_3J$, 60 g Essigsäure und 120 g N-Methyl-3-picoliniumjodid werden mit CO bei 60 bar und 450 K in einem Hastelloy-Autoklaven umgesetzt. Nach 32 min Reaktionszeit ergibt die Analyse 276 g Essigsäureanhydrid, entsprechend 828 g $Ac_2O$/g Rh.h.

Beispiel 3

250 g Methylacetat, 0,2 g $RhCl_3 \cdot 3H_2O$, 140 g $CH_3J$, 80 g Essigsäure und 180 g N-Methyl-3-picoliniumjodid werden mit CO bei 50 bar und 450 K in einem Hastelloy-Autoklaven umgesetzt. Nach einer Reaktionszeit von 78 min ergibt die Analyse 271 g Essigsäureanhydrid, entsprechend 2 667 g $Ac_2O$/g Rh.h.

Beispiel 4

250 g Methylacetat, 1,6 g $RhCl_3 \cdot 3H_2O$, 80 g $CH_3J$, 50 g Essigsäure und 100 g N-Methylchinoliniumjodid werden mit CO bei 35 bar und 455 K in einem Hastelloy-Autoklaven umgesetzt. Nach einer Reaktionszeit von 29 min werden 278 g Essigsäureanhydrid erhalten, entsprechend 919 g $Ac_2O$/g Rh.h.

Beispiel 5

250 g Methylacetat, 1,6 g $RhCl_3 \cdot 3H_2O$, 60 g $CH_3J$, 70 g Essigsäure und 70 g N,N-Dimethylimidazoliumjodid werden in einem Hastelloy-Autoklaven mit CO bei 50 bar und 450 K umgesetzt. Nach einer Reaktionszeit von 31 min werden im Reaktionsgemisch 283 g Essigsäureanhydrid gefunden, entsprechend 876 g $Ac_2O/g$ Rh.h.

Beispiel 6

250 g Methylacetat, 1,6 g $RhCl_3 \cdot 3H_2O$, 80 g $CH_3J$, 100 g Essigsäure und 180 g N,N-Dimethylimidazoliumjodid werden in einem Hastelloy-Autoklaven mit CO bei 30 bar und 443 K umgesetzt. Nach einer Reaktionszeit von 24 min werden 273 g Essigsäureanhydrid festgestellt, entsprechend 1 092 g $Ac_2O/g$ Rh.h.

Beispiel 7

250 g Methylacetat, 1,5 g $RhCl_3 \cdot 3H_2O$, 60 g $Ch_3J$, 60 g Essigsäure und 60 g N,N-Dimethylimidazoliumjodid werden bei 450 K und 50 bar in einem Hastelloy-Autoklaven mit einem Gemisch aus CO und $H_2$, das 8 Vol. % $H_2$ enthält, umgesetzt. Es werden nach 34 min 279 g Essigsäureanhydrid im Reaktionsgemisch ermittelt, entsprechend 840 g $Ac_2O/g$ Rh.h. Äthylidendiacetat konnte nur in Spuren nachgewiesen werden.

Beispiel 8

250 g Methylacetat, 2 g $Pd(CH_3CO_2)_2$, 40 g $CH_3J$, 80 g Essigsäure und 50 g N,N-Dimethylimidazoliumjodid werden in einem Hastelloy-Autoklaven bei 460 K und 50 bar mit CO umgesetzt. Nach 132 min ergibt die Analyse 191 g Essigsäureanhydrid, entsprechend 91 g $Ac_2O/g$ Pd.h.

Beispiel 9

250 g Methylacetat, 2 g $RhCl_3 \cdot 3H_2O$, 15 g $CH_3J$, 10 g Essigsäure und 30 g N-Methyl-3,4-lutiniumjodid werden mit CO bei 150 bar und 455 K in einem Hastelloy-Autoklaven umgesetzt. Nach 135 min ergibt die Analyse 259 g Essigsäureanhydrid, entsprechend 147 g $Ac_2O/g$ Rh.h.

Beispiel 10

250 g Methylacetat, 1,8 g $IrCl_3$, 50 g $CH_3J$, 60 g Essigsäure und 80 g N-Methyl-2,4-lutiniumjodid werden mit CO bei 120 bar und 470 K in einem Hastelloy-Autoklaven umgesetzt. Nach 84 min ergibt die Analyse 269 g Essigsäureanhydrid, entsprechend 166 g $Ac_2O/g$ Ir.h.

Beispiel 11

250 g Methylacetat, 1,6 g $RhCl_3 \cdot 3H_2O$, 50 g $CH_3J$, 50 g Essigsäure, 20 g N-Methylpyridiniumjodid und 40 g N-Methyl-3-picoliniumjodid werden in einem Hastelloy-Autoklaven bei 450 K und 30 bar mit CO umgesetzt. Nach einer Reaktionszeit von 39 min werden im Reaktionsgemisch 276 g Essigsäureanhydrid gefunden, ensprechend 679 g $Ac_2O/g$ Rh.h.

Beispiel 12

250 g Methylacetat, 1 g $RhCl_3 \cdot 3H_2O$, 50 g $CH_3J$, 50 g Essigsäure, 15 g N-Methylpyridiniumjodid und 30 g N-Methyl-2,4-lutidiniumjodid werden bei 430 K und 50 bar im Hastelloy-Autoklaven mit CO umgesetzt. Nach 170 min werden 269 g Essigsäureanhydrid analytisch festgestellt, entsprechend 243 g $Ac_2O/g$ Rh.h.

Beispiel 13

250 g Methylacetat, 0,8 g $RhCl_3 \cdot 3H_2O$, 100 g $CH_3J$, 90 g Essigsäure, 120 g N,N-Dimethylimidazoliumjodid und 60 g N-Methyl-3-picoliniumjodid werden in einem Hastelloy-Autoklaven mit CO bei 100 bar und 445 K umgesetzt. Nach einer Reaktionszeit von 38 min werden 278 g Essigsäureanhydrid festgestellt, entsprechend 1 402 g $Ac_2O/g$ Rh-h.

Beispiel 14

250 g Methylacetat, 1,6 g $RhCl_3 \cdot 3H_2O$, 60 g $CH_3J$, 50 g Essigsäure, 40 g Pyridiniumacetat werden bei 450 K und 50 bar mit CO im Hastelloy-Autoklaven umgesetzt. Nach 51 min Reaktionszeit werden 273 g Essigsäureanhydrid festgestellt, entsprechend 514 g $Ac_2O/g$ Rh.h.

Beispiel 15

250 g Methylacetat, 0,6 g $RhCl_3 \cdot 3H_2O$, 150 g $CH_3J$, 75 g Essigsäure und 100 g N-Methylimidazoliumacetat werden bei 460 K und 60 bar im Hastelloy-Autoklaven mit CO umgesetzt. Nach 71 min werden 281 g Essigsäureanhydrid, entsprechend 1 012 g $Ac_2O/g$ Rh.h ermittelt.

Beispiel 16

200 g Dimethyläther, 1,8 g $RhCl_3 \cdot 3H_2O$, 70 g $CH_3J$, 60 g Essigsäure und 90 g N-Methyl-3-picoliniumjodid werden bei 440 K und 80 bar mit CO im Hastelloy-Autoklaven umgesetzt. Nach einer Reaktionszeit von 126 min werden im methylacetathaltigen Reaktionsgemisch 209 g Essigsäureanhydrid festgestellt, entsprechend 141 g $Ac_2O/g$ Rh.h.

Beispiel 17

In einer kontinuierlichen Versuchsapparatur aus Hastelloy-Legierung werden dem mit 2 Liter Reaktionsgemisch gefüllten Reaktor stündlich 2,2 kg Frischmethylacetat zugeführt. Die mittlere Reaktionstemperatur beträgt 450 K. Der Reaktionsdruck wird durch kontinuierliche Zufuhr von CO auf 50 bar eingestellt. Das abfließende Reaktionsgemisch enthält ca. 9 Gew. % Methylacetat, ca. 55 Gew. % Acetanhydrid, ca. 10 Gew. % Essigsäure, ca. 10 Gew. % Methyljodid und ca. 13 Gew. % quaternäres Salz (Molverhältnis N-Methyl-3-picoliniumjodid zu N,N-Dimethylimidazoliumjodid = 1 :2). Die Rhodiumkonzentration

(RhCl$_3$ · 3H$_2$O) beträgt ca. 18 mMol Rh/l Reaktionsgemisch. Die destillative Aufarbeitung und Rückführung erfolgt in der im Fließschema beschriebenen Weise. Es werden stündlich ca. 3 kg Essigsäureanhydrid erhalten. Das entspricht einer Raumleistung von 1 500 g Ac$_2$O/l.h bzw. 811 g Ac$_2$O/g Rh.h. Die Anhydrid-Ausbeute, bezogen auf umgesetztes Methylacetat, ist nahezu quantitativ.

Beispiel 18

250 g Methylacetat, 1,6 g RhCl$_3$ · 3H$_2$O, 50 g Methyljodid, 40 g Ameisensäure und 60 g N,N-Dimethylimidazoliumjodid werden mit CO bei 50 bar und 450 K in einem Hastelloy-Autoklaven umgesetzt. Nach 68 min Reaktionszeit werden im Reaktionsprodukt 278 g Essigsäureanhydrid gefunden, entsprechend 392 g Ac$_2$O/g Rh.h.

Beispiel 19

250 g Methylacetat, 1,6 g RhCl$_3$ · 3H$_2$O, 50 g Methyljodid, 60 g Propionsäure und 60 g N,N-Dimethylimidazoliumjodid werden mit CO bei 50 bar und 450 K in einem Hastelloy-Autoklaven umgesetzt. Nach 52 min Reaktionszeit ergibt die Analyse die Bildung von 274 g Essigsäureanhydrid, entsprechend 505 g Ac$_2$O/g Rh.h.

Beispiel 20

250 g Methylacetat, 1,6 g RhCl$_3$ · 3H$_2$O, 50 g Methyljodid, 75 g Buttersäure und 60 g N,N-Dimethylimidazoliumjodid werden in einem Hastelloy-Autoklaven mit CO bei 60 bar und 450 K umgesetzt. Nach 58 min Reaktionszeit werden im Reaktionsprodukt 270 g Essigsäureanhydrid nachgewiesen, entsprechend 446 g Ac$_2$O/g Rh.h.

**Patentansprüche**

1. Verfahren zur Herstellung von Essigsäureanhydrid durch Umsetzung von Methylacetat und/oder Dimethyläther mit Kohlenmonoxid unter praktisch wasserfreien Bedingungen bei Temperaturen von 350 bis 575 K und Drücken von 1 bis 300 bar in Gegenwart eines Katalysatorsystems aus Edelmetallen der Gruppe VIII des Periodensystems der Elemente oder deren Verbindungen und Jod und/oder dessen Verbindungen sowie in Gegenwart einer aliphatischen Carbonsäure mit 1-8 Kohlenstoffatomen, dadurch gekennzeichnet, daß man als Promotor ausschließlich heterocyclische aromatische Verbindungen, in denen mindestens ein Heteroatom ein quaternäres Stickstoffatom ist und deren Schmelz- oder Mischschmelzpunkt unterhalb 413 K, dem Siedepunkt von Essigsäureanhydrid, liegt, einsetzt.

2. Verfahren nach Anspruch 1, dadurch gekennzeichnet, daß man die heterocyclischen Verbindungen in Form ihrer Addukte mit Essigsäure oder Methyljodid einsetzt.

3. Verfahren nach einem der vorhergehenden Ansprüche, dadurch gekennzeichnet, daß man das Katalysator- und Promotorsystem Edelmetall (verbindung)/Jod (-verbindung)/Carbonsäure/heterocyclische Verbindung im Atom- bzw. Molverhältnis 1 : (1-1 400) : (10-2 000) : (1-1 200) einsetzt.

4. Verfahren nach einem der vorhergehenden Ansprüche, dadurch gekennzeichnet, daß man Kohlenmonoxid/Wasserstoffgemische mit bis zu 10 Volum % Wasserstoff einsetzt.

**Claims**

1. Process for making acetic anhydride by reacting methyl acetate and/or dimethylether with carbon monoxide under practically anhydrous conditions, at temperatures of 350 to 575 K and under pressures of 1 to 300 bars in the presence of a catalyst system comprised of noble metals belonging to group VIII of the periodic system of the elements or their compounds and iodine and/or its compounds, and in the presence of an aliphatic carboxylic acid with 1 to 8 carbon atoms, characterized in that heterocyclic aromatic compounds, in which at least one hetero atom is a quaternary nitrogen atom and which have a melting point or mixed melting point of less than 413 K, this being the boiling point of acetic anhydride, are exclusively used as a promoter.

2. Process as claimed in claim 1, wherein the heterocyclic compounds are used in the form of their addition products with acetic acid or methyl iodide.

3. Process as claimed in any of the preceding claims, wherein the catalyst- and promoter-system comprised of noble metal (compound)/iodine (compound)/carboxylic acid/heterocyclic compound is used in the atomic or molar ratio of 1 : (1-1 400) : (10-2 000) : (1-1 200).

4. Process as claimed in any of the preceding claims, wherein carbon monoxide/hydrogen-mixtures containing up to 10 volume % of hydrogen are used.

**Revendications**

1. Procédé pour la préparation d'anhydride acétique par réaction d'acétate de méthyle et/ou d'éther diméthylique avec le monoxyde de carbone en conditions pratiquement anhydres, à des températures de 350 à 575 K et sous des pressions de 1 à 300 bars, en présence d'un système catalytique à base de métaux nobles du groupe VIII de la Classification Périodique des Eléments ou de leurs composés, et d'iode et/ou de ses composés, en présence d'un acide carboxylique aliphatique en C$_1$-C$_8$, caractérisé en ce que l'on utilise exclusivement comme activeur des composés aromatiques hétérocycliques, dans lesquels au moins un hétéroatome est un atome d'azote quaternaire et dont le point de fusion ou le point de fusion mixte est inférieur à 413 K, point d'ébullition de l'anhydride acétique.

2. Procédé selon la revendication 1, caractérisé en ce que l'on utilise les composés hétérocycliques sous forme de leurs adducts avec l'acide acétique ou l'iodure de méthyle.

3. Procédé selon l'une des revendications précédentes, caractérisé en ce que l'on utilise le système catalytique et activeur (composé de) métal noble/(composé d') iode/acide carboxylique/composé hétérocyclique dans un rapport atomique ou molaire de 1 : (1-1 400) : (10-2 000) : (1 : 1 200).

4. Procédé selon l'une des revendications précédentes, caractérisé en ce que l'on utilise des mélanges monoxydes de carbone/hydrogène contenant jusqu'à 10 % en volume d'hydrogène.